# EUROPEAN PATENT SPECIFICATION

(11) **EP 1 567 005 B1**
(45) Date of publication and mention of the grant of the patent: **20.02.2008**
(21) Application number: 03758346.5
(22) Date of filing: 22.10.2003
(51) Int. Cl.: A01N 39/04, C07C 235/20

(54) **FUNGICIDES**
FUNGIZIDE
FONGICIDES

(30) Priority: 26.11.2002 GB 0227558
(43) Date of publication of application: 31.08.2005
(73) Proprietor: Syngenta Limited, Guildford Surrey GU2 7YH (GB)
(72) Inventor: SALMON, Roger, Bracknell, Berks RG42 6EY (GB)
(74) Representative: Arunasalam, Velautha-Cumaran
(86) International application number: PCT/GB2003/004565
(87) International publication number: WO 2004/047537

(56) References cited:
- EP-A- 0 001 721

## Description

This invention relates to the use as plant fungicides of particular *N*-alkynyl-2-(substituted phenoxy)alkylamides. It also relates to plant fungicidal compositions containing these compounds and to some of the compounds themselves.

Certain *N*-alkynyl-2-(substituted phenoxy)alkylamides are described in US 4,116,677 and US 4,070,486 as being useful as herbicides or miticides. Others are described in US 4,168,319 as being useful as mildewicides.

The present invention is concerned with the provision of particular *N*-alkynyl-2-(substituted phenoxy)alkylamides for use as plant fungicides.

Thus according to the present invention there is provided the use as a plant fungicide of a compound of the general formula (1): 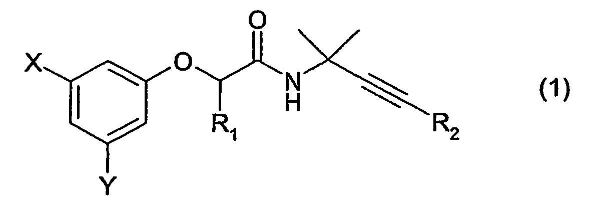 wherein X and Y are both chloro, bromo or methyl or X is methoxy and Y is cyano; R₁ is ethyl or *n*-propyl and R₂ is methyl or ethyl.

The compounds of the general formula (1) contain an asymmetric carbon atom and may exist as enantiomers or as mixtures of such. However, these mixtures may be separated into individual isomers, and this invention embraces such isomers and mixtures thereof in all proportions. It is to be expected that for any given compound, one isomer may be more fungicidally active than the other.

Of particular interest as plant fungicides are compounds where R₁ is ethyl and R₂ is methyl and compounds where R₁ and R₂ are both ethyl.

The invention also includes those compounds of general formula (1). Thus, in another aspect the invention provides a compound of the general formula (1) wherein X and Y are both chloro, bromo or methyl or X is methoxy and Y is cyano; R₁ is ethyl or *n*-propyl and R₂ is methyl or ethyl; provided that X and Y are not both chloro or methyl when R₁ is ethyl.

In yet another aspect the invention provides a compound of the general formula (1) wherein X and Y are both chloro or methyl; R₁ is *n*-propyl and R₂ is methyl or ethyl.

In yet another aspect the invention provides a compound of the general formula (1) wherein X and Y are both bromo or X is methoxy and Y is cyano; R₁is ethyl or *n-*propyl and R₂ is methyl or ethyl.

Compounds that may be used in the invention are illustrated in Table 1 below. The compounds have the general formula (1) with the values of X, Y, R₁ and R₂ given in the table.

Compounds 2, 4 to 8, 10 and 12 to 16 in Table 1 form part of the invention.

**TABLE 1**

| Compound | X | Y | R₁ | R₂ | Melting |
|---|---|---|---|---|---|
| No | | | | | Point (°C) |
| 1 | Cl | Cl | C₂H₅ | CH₃ | 99-103 |
| 2 | Br | Br | C₂H₅ | CH₃ | 115-117 |
| 3 | CH₃ | CH₃ | C₂H₅ | CH₃ | 68-71 |
| 4 | CH₃O | CN | C₂H₅ | CH₃ | |
| 5 | Cl | Cl | *n*-C₃H₇ | CH₃ | |
| 6 | Br | Br | *n*-C₃H₇ | CH₃ | |
| 7 | CH₃ | CH₃ | *n*-C₃H₇ | CH₃ | |
| 8 | CH₃O | CN | *n*-C₃H₇ | CH₃ | |
| 9 | Cl | Cl | C₂H₅ | C₂H₅ | 112.5-115 |
| 10 | Br | Br | C₂H₅ | C₂H₅ | |
| 11 | CH₃ | CH₃ | C₂H₅ | C₂H₅ | 94-96 |
| 12 | CH₃O | CN | C₂H₅ | C₂H₅ | |
| 13 | Cl | Cl | *n*-C₃H₇ | C₂H₅ | |
| 14 | Br | Br | *n*-C₃H₇ | C₂H₅ | |
| 15 | CH₃ | CH₃ | *n*-C₃H₇ | C₂H₅ | |
| 16 | CH₃O | CN | *n*-C₃H₇ | C₂H₅ | |

The compounds of formula (1) may be prepared as outlined in Schemes 1 to 6 below in which X, Y, R₁ and R₂ have the meanings given above, L is a leaving group such as a halide, for example iodide, or an alkyl or aryl sulphonyloxy group, for example methylsulphonyloxy and tosyloxy, Hal is halogen and R has the meaning ascribed to it in the text.

As shown in Scheme 1, the compounds of the general formula (1) may be prepared by reacting a phenol of the general formula (2) with a compound of the general formula (3) in the presence of a base in a suitable solvent. Typical solvents include *N,N-*dimethylformamide and *N*-methylpyrrolidin-2-one. Suitable bases include potassium carbonate, sodium hydride or di*iso*propylethylamine. Phenols of the general formula (2) are either commercially available or are known in the literature or may be prepared from known compounds by standard procedures.

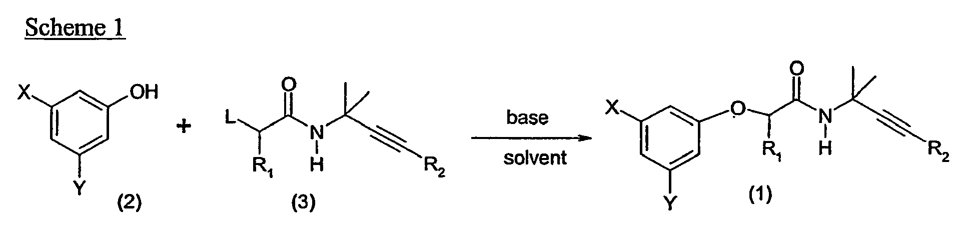

Compounds of the general formula (3) may be prepared as shown in Scheme 2 by reacting an amine (5) with an acid halide (4), or the corresponding acid anhydride, in the presence of a suitable inorganic or organic base, such as potassium carbonate or di*iso*propylethylamine and in a solvent such as dichloromethane or tetrahydrofuran.

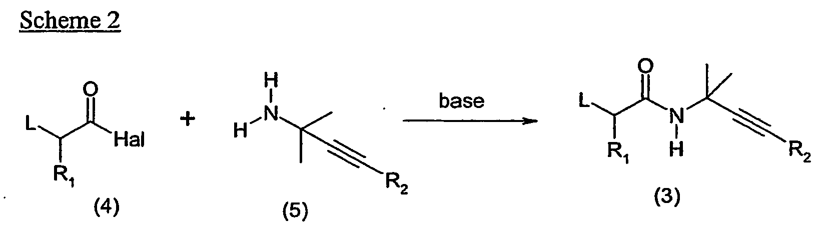

Amines of the general formula (5), wherein R₂ is methyl or ethyl, may be prepared as shown in Scheme 3 by alkylation of the silyl-protected aminoalkyne (7) using a suitable base, such as *n*-butyl lithium, followed by reaction with a suitable alkylating reagent R₂L, for example methyl or ethyl iodide, to form the alkylated compounds of general formula (8). 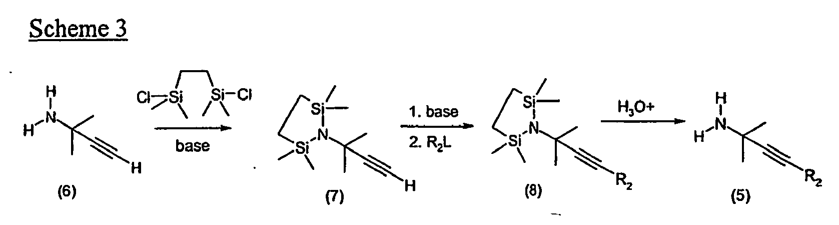 The silyl-protected aminoalkyne (7) may be obtained by reacting the amine (6) with 1,2-*bis*-(chlorodimethylsilyl)ethane in the presence of a suitable base, such as a tertiary organic amine base, for example triethylamine, in a suitable solvent, such as dichloromethane. The amine (6) is commercially available or may be prepared by standard literature methods (see, for example, EP-A-0834498).

Alternatively, as shown in Scheme 4, compounds of the general formula (1) may be prepared by condensing a compound of the formula (11), wherein R is hydrogen, with an amine of the general formula (5) using suitable activating reagents such as 1-hydroxybenzotriazole and *N*-(3-dimethylaminopropyl)-*N*'-ethyl-carbodiimide hydrochloride.

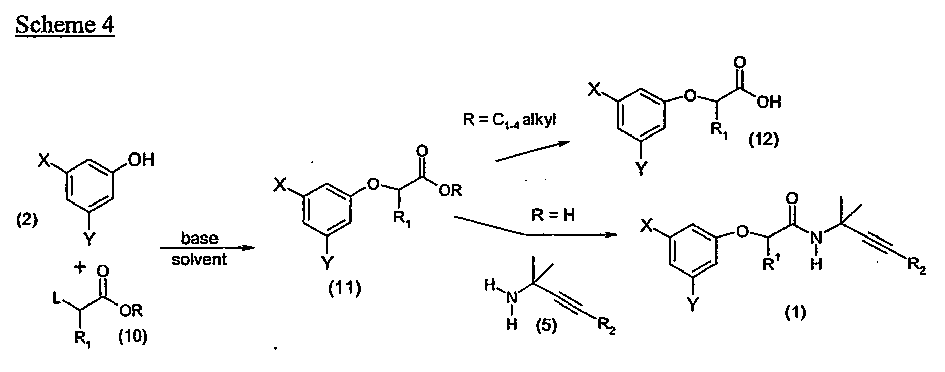

The acids of the general formula (12) may be prepared by the hydrolysis of the corresponding esters of the general formula (11), wherein R is C₁₋₄ alkyl, using known techniques. The esters of the general formula (11), wherein R is C₁₋₄ alkyl, and also the acids of the general formula (11), wherein R is hydrogen, may be prepared by reacting a phenol of the general formula (2) with an ester or acid of the general formula (10) in the presence of a suitable base, such as potassium carbonate or sodium hydride, in a suitable solvent, such as *N,N*-dimethylformamide. The ester or acid of the general formula (10) is either commercially available or may be prepared by standard literature methods from commercially available materials.

In another method shown in Scheme 5, the compounds of the general formula (1) may be prepared by reacting an acid halide of the general formula (13) with the amine of the general formula (5) in a suitable solvent, such as dichloromethane, in the presence of a tertiary amine, such as triethylamine, and an activating agent, such as 4-dimethylaminopyridine.

The acid halides of the general formula (13) may be prepared by chlorinating a compound of the general formula (12) with a suitable chlorinating agent, such as oxalyl chloride, in a suitable solvent, such as dichloromethane, and in the presence of, for example, *N,N*-dimethylformamide. The compounds of the general formula (12) correspond to the compounds of formula (11) where R is hydrogen.

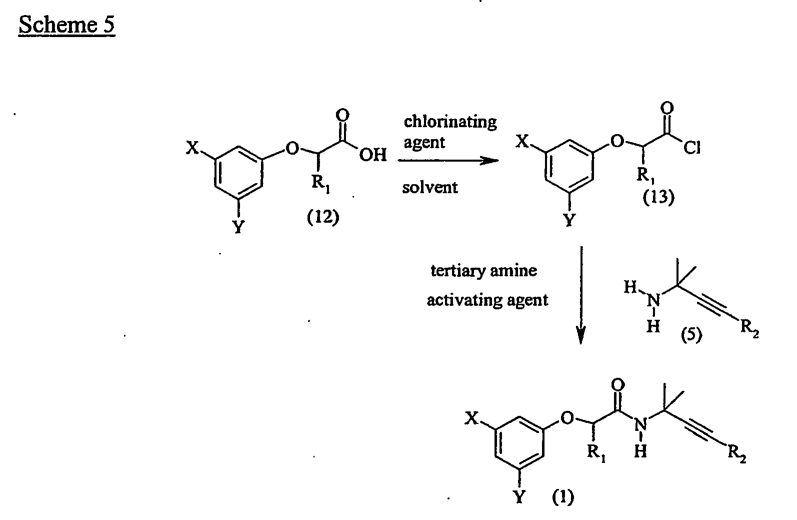

Alternatively, as shown in Scheme 6, compounds of the general formula (11), wherein R is C₁₋₄ alkyl, may be prepared under Mitsunobu conditions by reacting a phenol of general formula (2) with a compound of the general formula (14) using a phosphine, such as triphenyl phosphine, and an azoester, such as diethyl azodicarboxylate.

Similarly, compounds of general formula (1) may be prepared by reacting a compound of general formula (16) with a phenol of general formula (2) under Mitsunobu conditions using a phosphine, such as triphenyl phosphine, and an azoester, such as diethyl azodicarboxylate. Compounds of general formula (16) may be prepared from a compound of general formula (15) and an amine of general formula (5) using suitable activating reagents such as 1-hydroxybenzotriazole and *N*-(3-dimethylaminopropyl)-*N*'-ethyl-carbodiimide hydrochloride. Compounds (14) and (15) are either known compounds or may be made from known compounds.

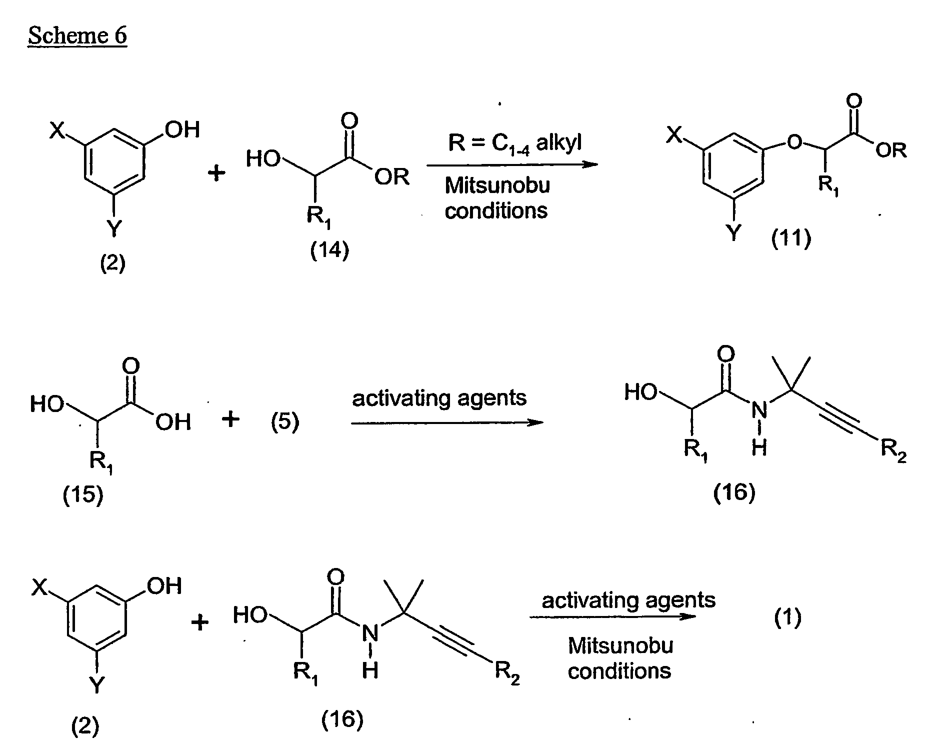

The compounds of formula (1) are active fungicides and may be used to control one or more of the following pathogens: *Pyricularia oryzae* (*Magnaporthe grisea*) on rice and wheat and other *Pyricularia* spp. on other hosts; *Puccinia triticina* (or *recondita*), *Puccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca (Sphaerotheca fuliginea)* on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. (*Pyrenophora* spp.), *Rhynchosporium* spp., *Mycosphaerella graminicola* (*Septoria tritici)* and *Phaeosphaeria nodorum* (*Stagonospora nodorum* or *Septoria nodorum*), *Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, -vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (*Glomerella cingulata*), black rot or frogeye leaf spot (*Botryosphaeria obtusa*), Brooks fruit spot (*Mycosphaerella pomi*), Cedar apple rust (*Gymnosporangium juniperi-virginianae*), sooty blotch (*Gloeodes pomigena*), flyspeck (*Schizothyrium pomi*) and white rot (*Botryosphaeria dothidea*)) on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum*) on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts,'vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum*; other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans*; and fungal vectors of viral diseases (for example *Polymyxa graminis* on cereals as the vector of barley yellow mosaic virus (BYMV) and *Polymyxa betae* on sugar beet as the vector of rhizomania).

The compounds of formula (1) show particularly good activity against the Oomycete class of pathogens such as *Phytophthora infestans, Plasmopara* species, e.g. *Plasmopara viticola* and *Pythium* species e.g. *Pythium ultimum.*

A compound of formula (1) may move acropetally, basipetally or locally in plant tissue to be active against one or more fungi. Moreover, a compound of formula (1) may be volatile enough to be active in the vapour phase against one or more fungi on the plant.

The invention therefore provides a method of combating or controlling phytopathogenic fungi which comprises applying a fungicidally effective amount of a compound of formula (1), or a composition containing a compound of formula (1), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The compounds of formula (1) are preferably used for agricultural, horticultural and turfgrass purposes in the form of a composition.

In order to apply a compound of formula (1) to a plant, to a seed of a plant, to the locus of the plant or seed or to soil or any other growth medium, a compound of formula (1) is usually formulated into a composition which includes, in addition to the compound of formula (1), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (1). The composition is generally used for the control of fungi such that a compound of formula (1) is applied at a rate of from 0.1g to 10kg per hectare, preferably from 1g to 6kg per hectare, more preferably from 1g to 1kg per hectare.

When used in a seed dressing, a compound of formula (1) is used at a rate of 0.0001 g to 10g (for example 0.001 g or 0.05 g), preferably 0.005 g to 10g, more preferably 0.005 g to 4g, per kilogram of seed.

In another aspect the present invention provides a fungicidal composition comprising a fungicidally effective amount of a compound of formula (1) and a suitable carrier or diluent therefor.

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (1).

Dustable powders (DP) may be prepared by mixing a compound of formula (1) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (1) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (1) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (1) and one or more powdered solid diluents or carriers, or from pre-formed blank granules by absorbing a compound of formula (1) (or a solution thereof, in a suitable agent) in a porous granular material (such as pumice, attapulgite clays, fuller's earth, kieselguhr, diatomaceous earths or ground corn cobs) or by adsorbing a compound of formula (1) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (1) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (1) in an organic solvent (optionally containing one or more wetting agents, one or more emulsifying agents or a mixture of said agents). Suitable organic solvents for use in ECs include aromatic hydrocarbons (such as alkylbenzenes or akylnaphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (1) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents that have a low solubility in water.

Microemulsions (ME) may be prepared by mixing water with a blend of one or more solvents with one or more SFAs, to produce spontaneously a thermodynamically stable isotropic liquid formulation. A compound of formula (1) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (1). SCs may be prepared by ball or bead milling the solid compound of formula (1) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (1) may be dry milled and added to water, containing agents hereinbefore described, to produce the desired end product.

Aerosol formulations comprise a compound of formula (1) and a suitable propellant (for example *n*-butane). A compound of formula (1) may also be dissolved or dispersed in a suitable medium (for example water or a water miscible liquid, such as *n-*propanol) to provide compositions for use in non-pressurised, hand-actuated spray pumps.

A compound of formula (1) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (1) and, optionally, a carrier or diluent therefor. The polymeric shell may be produced by either an interfacial polycondensation reaction or by a coacervation procedure. The compositions may provide for controlled release of the compound of formula (1) and they may be used for seed treatment. A compound of formula (1) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (1)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (1)).

A compound of formula (1) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (1) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (1) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (1) (for example 0.0001 to 10%, by weight) depending upon the purpose for which they are to be used.

A compound of formula (1) may be used in mixtures with fertilisers (for example nitrogen-, potassium- or phosphorus-containing fertilisers). Suitable formulation types include granules of fertiliser. The mixtures suitably contain up to 25% by weight of the compound of formula (1).

The invention therefore also provides a fertiliser composition comprising a fertiliser and a compound of formula (1).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (1) alone. Further the other fungicide may have a synergistic effect on the fungicidal activity of the compound of formula (1).

The compound of formula (1) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (1); or help to overcome or prevent the development of resistance to individual components. The particular additional active ingredient will depend upon the intended utility of the composition.

Examples of fungicidal compounds which may be included in the composition of the invention are AC 382042 (*N*-(1-cyano-1,2-dimethylpropyl)- 2-(2,4-dichlorophenoxy) propionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan, carbendazim, carbendazim chlorhydrate, carboxin, carpropamid, carvone, CGA 4 1396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, *O,O*-di-*iso*-propyl-S-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (*Z*)-*N*-benzyl-*N*([methyl(methyl-thioethylideneaminooxycarbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (N allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyanomethylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin, XRD-563, zineb, ziram, zoxamide and compounds of the formulae:

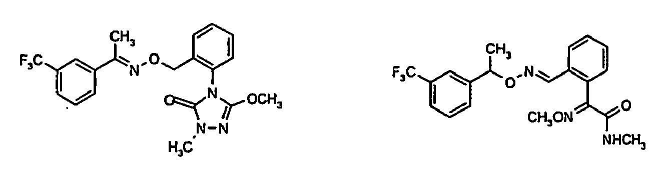

The compounds of formula (1) may be mixed with soil, peat or other rooting media for the protection of plants against seed-bome, soil-borne or foliar fungal diseases.

Some mixtures may comprise active ingredients that have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples in which the following abbreviations are used:

| | |
|---|---|
| ml = millilitres | DMSO = dimethylsulphoxide |
| g = grammes | NMR = nuclear magnetic resonance |
| ppm = parts per million | d = doublet |
| m.p. = melting point | t = triplet |
| b.p. = boiling point | q = quartet |
| s = singlet | m = multiplet |
| br s = broad singlet | |

### EXAMPLE 1

This Example illustrates the preparation of 2-(3-cyano-5-methoxyphenoxy)-*N*-(4-methylpent-2-yn-4-yl)butyramide (Compound No. 4 in Table 1)

3-Cyano-5-methoxyphenol (prepared as described in J. Med. Chem. (1993), 36, N°16, 2367; 0.149g) in dry *N*,*N*-dimethylformamide (5ml) containing anhydrous potassium carbonate (0.140g) and 2-bromo-*N*-(4-methylpent-2-yn-4-yl)butyramide (0.246g) were stirred and heated to 80°C for 7 hours. The mixture was cooled to ambient temperature, stored for 18 hours then neutralised with dilute aqueous hydrochloric acid. The reaction mixture was diluted with water, extracted with diethyl ether and the organic phase was separated, washed with brine, dried over magnesium sulphate then evaporated under reduced pressure to give a brown gum. The gum was fractionated by chromatography (silica; hexane/ethyl acetate, 5-50%) to give a colourless gum that was triturated with diethyl ether/hexane to give the required product as a colourless solid (0.134g) m.p. 97-98°C.
¹H NMR (CDCl₃) δ: 0.94(3H, s); 1.52 (6H, s); 1.72(3H, s); 1.89(2H, m); 3.74(3H, s); 4.32(1H, t); 6.22(1H, s); 6.61(1H, m); 6.72(1H, m); 6.76(1H, m).

### Preparation of 2-bromo-N-(4-methylpent-2-yn-4-yl)butyramide.

4-Amino-4-methylpent-2-yne hydrochloride (S.Og).was dissolved in dry dichloromethane (200ml) and cooled to 3°C with stirring. To the mixture was added 2-bromobutyryl bromide (6.25g) followed by dropwise addition of dry triethylamine (10.93ml), maintaining the reaction at 5°C during the addition by cooling. The suspension, which had formed during the reaction, was stirred at ambient temperature for 1 hour then water was added. The organic phase was separated, washed with water, dried over magnesium sulphate then evaporated under reduced pressure. The residue was fractionated by chromatography (silica; hexane/diethyl ether, 3:1 by volume) to give the required product (5.2g) as a colourless solid.
¹H NMR (CDCl₃) δ: 1.04(3H, t); 1.64(6H, s); 1.84(3H, s); 2.04-2.18(2H, m); 4.20-4.24(1H, m); 6.46(1H, br s).

### Preparation of 4-amino-4-methylpent-2-yne hydrochloride

### Stage 1

3-Amino-3-methylbutyne (commercially available as 90% aqueous solution) was dissolved in dichloromethane (150ml), dried over sodium sulphate and filtered to give a solution containing 16.6g of amine. To the stirred solution of amine under an atmosphere of nitrogen at ambient temperature was added dry triethylamine (48.4ml). Then 1,2-*bis-*(chlorodimethylsilyl)ethane (38.98g) in dichloromethane (100ml) was added dropwise, the reaction temperature being maintained at 15°C by cooling. The mixture was stirred for 3 hours, the colourless solid, which had formed during the reaction, was filtered from solution and the filtrate was evaporated under reduced pressure to give a paste. The paste was extracted into hexane and refiltered. The filtrate was evaporated under reduced pressure and the oil obtained was distilled to give 1-(1,1-dimethyl-2-propynyl)-2,2,5,5-tetramethyl-1-aza-2,5-disilacyclopentane (21.5g) b.p. 41°C at 0.06 mm Hg.
¹H NMR (CDCl₃) δ: 0.16(12H, s); 0.60(4H,s); 1.48(6H, s); 2.24(1H, s).

### Stage 2

The product from Stage 1 (13.0g) in dry tetrahydrofuran (140ml) was cooled to -70°C under an atmosphere of nitrogen with stirring and a solution of *n*-butyl lithium (23.1ml of 2.5M solution in hexanes) was added at -(65-70)°C during 5 minutes. The mixture was allowed to warm to -5°C and methyl iodide (3.93ml) was added dropwise over 10 minutes. The reaction mixture was allowed to warm to 10°C when an exothermic reaction occurred. The mixture was maintained at 20°C by cooling for 2 hours then evaporated under reduced pressure to a small volume. The residue was dissolved in hexane, filtered to remove the insoluble material and evaporated under reduced pressure to give 1-(1,1-dimethyl-2-butynyl)-2,2,5,5-tetramethyl-1-aza-2,5-disilacyclopentane as a yellow oil (13.0g).
¹H NMR (CDCl₃) δ: 0.10(12H,s); 0.56(4H, s); 1.40(6H, s); 1.72(3H, s).

### Stage 3

The product from Stage 2 (13.0g) was added slowly to aqueous hydrochloric acid (35ml, 4M) at 0°C with stirring. The emulsion formed was stirred for 0.5 hours then taken to pH14 with aqueous sodium hydroxide (4M) while maintaining the reaction mixture at 0°C by cooling in ice. The aqueous mixture was extracted into dichloromethane (three times). The extracts were combined, dried (sodium sulphate) and filtered. The filtrate was made acidic by adding an excess of a saturated solution of hydrogen chloride in 1,4-dioxan. The mixture was concentrated under reduced pressure until a colourless precipitate was formed. Hexane was added to the suspension then the solid was filtered from solution. The solid was washed with dry diethyl ether and placed under vacuum to remove any residual solvents to give the required product as a colourless solid (5.0g).
¹H NMR (d6-DMSO) δ:1.74(6H, s); 1.82(3H, s); 8.74(3H, br s).

### EXAMPLE 2

For the preparation of Compounds 9 to 16 in Table 1, 5-amino-5-methylhex-3-yne hydrochloride was prepared in a similar way to 4-amino-4-methylpent-2-yne hydrochloride, as described in Example 1, except that ethyl iodide was used in place of methyl iodide in Stage 2.
¹H NMR (d6-DMSO) δ: 1.10-1.16(3H, t); 1.74(6H, s); 2.16-2.20(2H, q); 8.70(3H, br s).

### EXAMPLE 3

### Preparation of (3,5-Dibromophenoxy)-N (4-methylpent-2-yn-4-yl)butyramide (Compound No. 2 in Table 1)

(3,5-Dibromophenoxy)-*N*-(4-methylpent-2-yn-4-yl)butyramide may be prepared according to the method described in Example II of US-A-4116677 for (3,5-dichlorophenoxy)-*N*-(4-methylpent-2-yn-4-yl)butyramide (Compound 1 in Table 1) except that 3,5-dibromophenol is used in place of 3,5-dichlorophenol.

### EXAMPLE 4

This Example illustrates the fungicidal properties of compounds of formula (1).

The compounds were tested in a leaf disk assay, with methods described below. The test compounds were dissolved in DMSO and diluted into water to 200 ppm. In the case of the test on *Pythium ultimum,* they were dissolved in DMSO and diluted into water to 20 ppm.
*Erysiphe graminis f.sp. hordei* (barley powdery mildew): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Erysiphe graminis f.sp. tritici* (wheat powdery mildew): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Puccinia recondita f.sp. tritici* (wheat brown rust): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed nine days after inoculation as preventive fungicidal activity. *Septoria nodorum* (wheat glume blotch): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Pyrenophora teres* (barley net blotch): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Pyricularia oryzae* (rice blast): Rice leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Botrytis cinerea* (grey mould): Bean leaf disks were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Phytophthora infestans* (late blight of potato on tomato): Tomato leaf disks were placed on water agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity. *Plasmopara viticola* (downy mildew of grapevine): Grapevine leaf disks were placed on agar in a 24-well plate and sprayed a solution of the test compound. After allowing to dry completely, for between 12 and 24 hours, the leaf disks were inoculated with a spore suspension of the fungus. After appropriate incubation the activity of a compound was assessed seven days after inoculation as preventive fungicidal activity.
*Pythium ultimum* (Damping off): Mycelial fragments of the fungus, prepared from a fresh liquid culture, were mixed into potato dextrose broth. A solution of the test compound in dimethyl sulphoxide was diluted with water to 20ppm then placed into a 96-well microtiter plate and the nutrient broth containing the fungal spores was added. The test plate was incubated at 24°C and the inhibition of growth was determined photometrically after 48 hours.
The following compounds (Compound No. (Table No.)) gave more than 60% control of the following fungal infections at 200ppm:
*Phytophthora infestans*: 1(1), 4(1), 9(1),
*Plasmopara viticola*: 1(1), 2(1), 3(1); 4(1), 9(1), 11(1)
*Erysiphe graminis f.sp tritici*: 1(1), 4(1)
The following compounds gave more than 60% control of the following fungal infection at 20ppm:
*Pythium ultimum*: 1(1), 3(1), 4(1), 11(1)

## Claims

1. The use as a plant fungicide of a compound of the general formula (1): 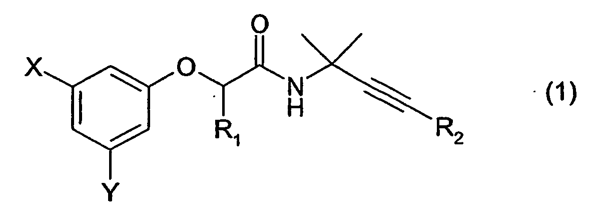 wherein X and Y are both chloro, bromo or methyl or X is methoxy and Y is cyano; R₁ is ethyl or *n*-propyl and R₂ is methyl or ethyl.

2. The use as a plant fungicide of a compound of the general formula (1) according to claim 1 wherein R₁ is ethyl and R₂ is methyl.

3. The use as a plant fungicide of a compound of the general formula (1) according to claim 1 wherein R₁ and R₂ are both ethyl.

4. A compound of the general formula (1) as defined in claim 1 wherein X and Y are both chloro, bromo or methyl or X is methoxy and Y is cyano; R₁ is ethyl or *n-*propyl and R₂ is methyl or ethyl; provided that X and Y are not both chloro or methyl when R₁ is ethyl.

5. A compound of the general formula (1) as defined in claim 1 wherein X and Y are both chloro or methyl; R₁ is *n*-propyl and R₂ is methyl or ethyl.

6. A compound of the general formula (1) as defined in claim 1 wherein X and Y are both bromo or X is methoxy and Y is cyano; R₁ is ethyl or *n*-propyl and R₂ is methyl or ethyl.

7. A fungicidal composition comprising a fungicidally effective amount of a compound of formula (1) as defined in claim 1 and a suitable carrier or diluent therefor.

8. A method of combating or controlling phytopathogenic fungi which comprises applying a fungicidally effective amount of a compound of formula (1) as defined in claim 1 or a composition according to claim 7 to a plant, to a seed of a plant, to the locus of the plant or seed or to soil or any other plant growth medium.

## Patentansprüche

1. Verwendung einer Verbindung der allgemeinen Formel (1): 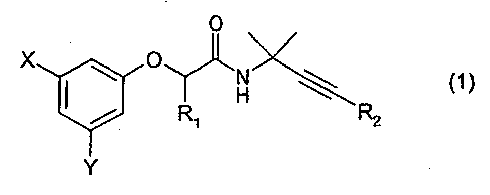 worin X und Y beide Chlor, Brom oder Methyl darstellen oder X Methoxy darstellt und Y Cyano darstellt; R₁ Ethyl oder n-Propyl darstellt und R₂ Methyl oder Ethyl darstellt, als ein Pflanzenfungizid.

2. Verwendung einer Verbindung der allgemeinen Formel (1) nach Anspruch 1, worin R₁ Ethyl darstellt und R₂ Methyl darstellt, als ein Pflanzenfungizid.

3. Verwendung einer Verbindung der allgemeinen Formel (1) nach Anspruch 1, worin R₁ und R₂ beide Ethyl darstellen, als ein Pflanzenfungizid.

4. Verbindung der allgemeinen Formel (1), wie in Anspruch 1 definiert, worin X und Y beide Chlor, Brom oder Methyl darstellen oder X Methoxy darstellt und Y Cyano darstellt; R₁ Ethyl oder n-Propyl darstellt und R₂ Methyl oder Ethyl darstellt; mit der Maßgabe, dass X und Y nicht beide Chlor oder Methyl darstellen, wenn R₁ Ethyl darstellt.

5. Verbindung der allgemeinen Formel (1), wie in Anspruch 1 definiert, worin X und Y beide Chlor oder Methyl darstellen; R₁ n-Propyl darstellt und R₂ Methyl oder Ethyl darstellt.

6. Verbindung der allgemeinen Formel (1), wie in Anspruch 1 definiert, worin X und Y beide Brom darstellen oder X Methoxy darstellt und Y Cyano darstellt; R₁ Ethyl oder n-Propyl darstellt und R₂ Methyl oder Ethyl darstellt.

7. Fungizide Zusammensetzung, umfassend eine fungizid wirksame Menge einer Verbindung der Formel (1) nach Anspruch 1, und ein geeignetes Träger- oder Verdünnungsmittel dafür.

8. Verfahren zum Bekämpfen oder Steuern bzw. Eindämmen von phytopathogenen Pilzen, das Applizieren einer fungizid wirksamen Menge einer Verbindung der Formel (1) nach Anspruch 1 oder einer Zusammensetzung nach Anspruch 7 auf eine Pflanze, auf ein Saatgut einer Pflanze, auf den Standort der Pflanze oder des Saatguts, oder den Boden oder ein beliebiges anderes Pflanzenwachstumsmedium umfasst.

## Revendications

1. Utilisation d'un fongicide pour plantes à base d'un composé de formule générale (1) : 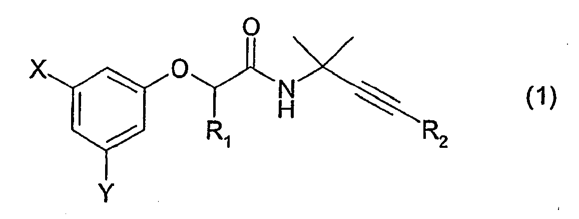 dans laquelle X et Y sont tous les deux un groupe chloro, bromo ou méthyle où X est un groupe méthoxy et Y est un groupe cyano ; R₁ est un groupe éthyle ou *n*-propyle et R₂ est un groupe méthyle ou éthyle.

2. Utilisation d'un fongicide pour plantes à base d'un composé de formule générale (1) selon la revendication 1, dans laquelle R₁ est un groupe éthyle et R₂ est un groupe méthyle.

3. Utilisation d'un fongicide pour plantes à base d'un composé de formule générale (1) selon la revendication 1, dans laquelle R₁ et R₂ sont tous les deux un groupe éthyle.

4. Composé de formule générale (1) tel que défini dans la revendication 1, dans lequel X et Y sont tous les deux un groupe chloro, bromo ou méthyle ou X est un groupe méthoxy et Y est un groupe cyano ; R₁ est un groupe éthyle ou *n-*propyle et R₂ est un groupe méthyle ou éthyle ; à condition que X et Y ne soient pas tous les deux un groupe chloro ou méthyle lorsque R₁ est un groupe éthyle.

5. Composé de formule générale (1) tel que défini dans la revendication 1, dans lequel X et Y sont tous les deux un groupe chloro ou méthyle ; R₁ est un groupe *n*-propyle et R₂ est un groupe méthyle ou éthyle.

6. Composé de formule générale (1) tel que défini dans la revendication 1, dans lequel X et Y sont tous les deux un groupe bromo ou X est un groupe méthoxy et Y est un groupe cyano ; R₁ est un groupe éthyle ou *n*-propyle et R₂ est un groupe méthyle ou éthyle.

7. Composition fongicide comprenant une quantité efficace sur le plan fongicide d'un composé de formule (1) tel que défini dans la revendication let un vecteur adapté ou un diluant pour celui-ci.

8. Procédé de lutte contre les champignons phytopathogènes qui comprend l'application d'une quantité efficace sur le plan fongicide d'un composé de formule (1) tel que défini dans la revendication 1, ou d'une composition selon la revendication 7 à une plante, à une graine d'une plante, à l'endroit où se trouve la plante ou la graine ou au sol ou à tout autre milieu de croissance végétal.
